Europäisches Patentamt

European Patent Office

Office européen des brevets

(19)

(11) Publication number: 0 385 631
A1

# EUROPEAN PATENT APPLICATION

(12)

(21) Application number: 90301767.1

(22) Date of filing: 19.02.90

(51) Int. Cl.5 **C07C 31/20, C07C 29/50**

(30) Priority: 21.02.89 US 312265

(43) Date of publication of application:
05.09.90 Bulletin 90/36

(84) Designated Contracting States:
BE DE ES FR GB IT NL

(71) Applicant: ARCO CHEMICAL TECHNOLOGY INC.
3 Christina Centre Suite 902 201 N Walnut Street
Wilmington Delaware 19801(US)

(72) Inventor: Gaffney, Anne M.
611 Owen Road
West Chester, PA 19380(US)
Inventor: Sofranko, John A.
119 Hedgerow Lane
West Chester, PA 19380(US)

(74) Representative: Cropp, John Anthony David et al
MATHYS & SQUIRE 10 Fleet Street
London, EC4Y 1AY(GB)

(54) Oxidation process.

(57) A process is provided for the conversion of an olefin to the corresponding glycol wherein a mixture of olefin, oxygen, carbon dioxide and water is reacted in a solvent at supercritical conditions with respect to the solvent.

EP 0 385 631 A1

## OXIDATION PROCESS

### Background of the Invention

#### Field of the Invention

The present invention relates to the oxidation of olefins such as propylene to the corresponding glycol wherein the oxidation is carried out in a solvent at supercritical conditions with respect to the solvent. Illustrative solvents are carbon dioxide and methane.

#### Description of the Prior Art

The catalytic oxidation of olefins to the corresponding glycols is a known reaction which is generally carried out in aqueous acidic media. See for example German patents 1948786 and 1948838.

Problems associated with such systems include the necessity for corrosion-resistant materials of construction and difficulties in product separation.

Olefin oxidation at supercritical conditions using carbon dioxide solvent and thallic oxide catalyst to produce the corresponding olefin oxide is known. See U.S. Patent 4,483,996.

Likewise, the oxidation of olefins to alkylene carbonates using a catalytic system consisting of iodine or iodide and an oxygen transfer material such as manganese dioxide is known. See U.S. Patent 4,009,183. In this latter patent, water is employed as solvent alone or in admixture with miscible polar solvent such as acetonitrile; a broad pressure range is disclosed but the examples use only 35 atm or less. Reaction temperatures are well below the critical temperatures of water and acetonitrile.

#### Brief Description of the Invention

In accordance with the present invention, olefins are oxidized to the corresponding glycol by reaction of olefin, oxygen, carbon dioxide and water in a solvent at conditions which are supercritical with respect to the solvent. Preferred solvents are carbon dioxide and methane. A catalyst system, preferably a heterogeneous solid system, is employed.

### Detailed Description of the Invention

The present invention is applicable generally to the conversion of olefins to the corresponding glycol. Preferably unsubstituted linear or cyclic olefins having 2 to 12 carbon atoms are converted. Examples include ethylene, propylene, the butenes, cyclohexene, 1-decene, 1-dodecene, cyclododecene, and the like. Propylene is the preferred olefin.

Molecular oxygen is provided as the oxidant. Air may be used as may pure oxygen or oxygen diluted with various inert gases.

Water is a necessary reagent and is provided in amount at least stoichiometrically equivalent to olefin which is converted, i.e., a mol of water per mol of olefin converted.

Carbon dioxide is also a necessary reagent. Where carbon dioxide solvent is employed, the solvent provides the necessary carbon dioxide for the olefin conversion. Where a different solvent is employed, e.g., methane, carbon dioxide reactant must be added.

While not intending to be bound by theory, it is believed that the olefin conversion first proceeds to the carbonate and, at the conditions employed, the carbonate reacts with water to form the product glycol. While carbon dioxide is essential, there is no net consumption of this material.

Elevated temperatures above the critical temperature of the solvent are necessary to carry out the reaction. Temperatures broadly ranging from about 70° C to 400° C, preferably 100° C to 250° C are employed.

A solvent is employed which forms a homogeneous reaction solution at conditions which are supercritical with respect to the solvent. Preferred solvents are carbon dioxide or methane. Materials which are reagents such as water or olefin can also function effectively as solvent. Mixtures of materials may be employed as the solvent. Conditions in the reaction zone must be maintained supercritical with respect to the solvent in accordance with the invention. Preferably pressures which are supercritical with respect to the solvent and which are in excess of 75 atmospheres are employed, especially pressures of 100 to 500 atmospheres.

Catalyst systems comprised of copper and iodine components together with an oxygen transfer agent are employed. A preferred catalyst system comprises CuI and Cu$_2$O with MnO$_2$ as oxygen transfer agent to facilitate reoxidation of cuprous components. For ease of product separation, the catalyst system is suitably supported on a solid such as MgO. An illustrative system is represented by CuI (5 wt. % as Cu), $Cu_2O$ (10 wt. % as Cu) and Na $MnO_4$ (12.5 wt. %) on MgO.

The following examples illustrate the invention.

## Example 1

A feed mixture comprised of 6.29 moles $CO_2$, 0.574 moles $O_2$, 0.487 moles propylene, 0.309 moles nitrogen, and 1.11 moles water was charged to a pressure reactor to which also was added 5.51 grams of a heterogeneous catalyst system comprising $CaI_2$, (5 wt. % as Ca), CuI (5 wt. % as Cu), $Cu_2O$ (10 wt. % as Cu) on $MnO_2$ support.

The catalyst was contained in a basket which was rotated during the reaction. The reaction was carried out for 10 hours at 146° C and 2217 psig.

About 3.8% of the $O_2$ was converted, selectivity to propylene glycol was 91.5%.

## Example 2

A feed mixture comprised of 0.0444 moles $O_2$, 0.167 moles $CO_2$, 0.487 moles propylene, 0.309 moles $N_2$, 1.215 moles methane and 1.11 moles water was charged to a Berty spinning basket reactor. About 7.33 grams of a heterogeneous catalyst system comprised of CuI (5 wt. % Cu), $Cu_2O$ (10 wt. % as Cu) and 12.5 wt. % Na $MnO_4$ on MgO was placed in the basket. Basket rotation was at 500 rpm during the reaction.

The reaction was carried out at 100° C and 1550 psig for 3 hours.

About 2.4% oxygen conversion was achieved with 69.3% selectivity to propylene glycol, 12.9% selectivity to acetone and 17.8% selectivity to propylene carbonate.

## Claims

1. The process for the catalytic conversion of an olefin to the corresponding glycol which comprises reacting the olefin, oxygen, carbon dioxide and water in the presence of a catalyst in a solvent at conditions which are supercritical with respect to the solvent.

2. The process of claim 1 wherein said olefin is propylene.

3. The process of claim 1 or claim 2 wherein said solvent is carbon dixoide.

4. The process of claim 1 or claim 2 wherein said solvent is methane.

5. The process of any one of claims 1 to 4 wherein the catalyst is a heterogeneous solid catalyst.

6. The process of any one of claims 1 to 5 wherein the conditions which are supercritical with respect to the solvent are a temperature in the range of 70 to 400° C and a pressure in excess of 75 atmospheres.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US-A-4 483 996 (JACOBSON) <br> * column 3, lines 6-59 * | 1-6 | C 07 C 31/20 <br> C 07 C 29/50 |
| Y | DD-A- 210 026 (HALCON) <br> * page 4, line 8 - page 6, line 17 * | 1-6 | |
| D,A | US-A-4 009 183 (FUMAGALLI) <br> * claims 1,5; column 2, lines 64,65 * | 1 | |
| A | GB-A-2 050 376 (ICI) <br> * claim 1 * | 1 | |
| A | CH-A- 580 553 (SOCIETA ITALIANA RESINE) <br> * claim 1 * | 1 | |
| A | CHEMICAL ABSTRACTS <br> vol. 107, no. 13, 28 September 1987, abstract no. 115290q, Columbus, Ohio, US; K.M. DOOLEY et al.: "Oxidation catalysis in a supercritical fluid medium" & Ind. Eng. Chem. Res. 1987, vol. 26, no. 9, pages 1910-1916 | 1 | |

TECHNICAL FIELDS SEARCHED (Int. Cl.5)

C 07 C 31/00
C 07 C 29/00

The present search report has been drawn up for all claims

| Place of search | Date of completion of the search | Examiner |
|---|---|---|
| BERLIN | 07-06-1990 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)